# EUROPÄISCHE PATENTSCHRIFT

(11) **EP 2 566 572 B1**
(45) Veröffentlichungstag und Bekanntmachung des Hinweises auf die Patenterteilung: **12.07.2017**
(21) Anmeldenummer: 11718300.4
(22) Anmeldetag: 06.05.2011
(51) Int. Cl.: A61N 1/05

(54) **ELEKTRODE UND VERFAHREN ZUR HERSTELLUNG EINER DERARTIGEN ELEKTRODE**
ELECTRODE AND PROCESS FOR FABRICATING SUCH AN ELECTRODE
ELECTRODE ET PROCEDE DE FABRICATION D'UNE TELLE ELECTRODE

(30) Priorität: 06.05.2010 DE 102010019648
(43) Veröffentlichungstag der Anmeldung: 13.03.2013
(73) Patentinhaber: Acandis GmbH & Co. KG, 76327 Pfinztal (DE)
(72) Erfinder: CATTANEO, Giorgio, 76199 Karlsruhe (DE)
(74) Vertreter: Kilchert, Jochen
(86) Internationale Anmeldenummer: PCT/EP2011/002263
(87) Internationale Veröffentlichungsnummer: WO 2011/138046

(56) Entgegenhaltungen:
- WO-A2-2007/146060
- US-A1- 2002 043 456
- US-A1- 2007 061 006

## Beschreibung

Die Erfindung betrifft eine Elektrode, insbesondere zur Messung elektrischer Aktivitäten eines lebenden Körpers und/oder zur Abgabe elektrischer Signale in den lebenden Körper mit den Merkmalen des Oberbegriffs des Anspruchs 1. Die Erfindung betrifft ferner ein Verfahren zur Herstellung einer derartigen Elektrode.

Eine Elektrode der eingangs genannten Art ist beispielsweise aus DE 199 30 271 A1 und aus WO 2007 / 146 060 A2 bekannt.

Elektroden werden im medizinischen Bereich sowohl zur Abgabe elektrischer Signale an den lebenden Körper als auch zur Messung elektrischer Aktivitäten von Körperzellen eingesetzt. Dabei kann die Messung bzw. die Stimulation von Körperzellen innerhalb des Körpers oder außerhalb des Körpers, beispielsweise auf der Hautoberfläche, erfolgen. Elektroden für medizinische Einsatzzwecke können für die dauerhafte Implantation, eine temporäre Implantation oder einen temporären Kontakt mit dem Gewebe vorgesehen sein. Beispielsweise ist es bekannt, Elektroden über einen Katheter an einen Messort zu führen und nach erfolgter Messung oder Stimulation zu entfernen. In allen Fällen besteht das Bedürfnis, eine möglichst hohe Flexibilität der Elektrode bereitzustellen, so dass die Behandlungs- bzw. Messorte in oder außerhalb des Körpers gut erreicht werden. Insbesondere ist es gewünscht, einen sicheren Kontakt zwischen der Elektrode und dem Körpergewebe herzustellen.

Die eingangs genannte, bekannte Elektrode weist eine Elektrodenspitze auf, die eine elektrisch leitende Oberfläche umfasst. Die Elektrodenspitze ist mit einer elektrisch isolierten Leitung verbunden. Im Gebrauch ist die Elektrodenspitze mit Körpergewebe in Kontakt, so dass elektrische Signale bzw. elektrische Impulse an das Körpergewebe übertragen werden können. Die bekannt Elektrode ist insbesondere für die Implantation im Herz vorgesehen, um elektrische Signale eines implantierten Schrittmachers oder Defibrillators an die Herzmuskelzellen zu übertragen. Die Verbindung zwischen der Vorrichtung, die die elektrischen Signale erzeugt, und der Elektrodenspitze, erfolgt über die elektrisch isolierte Leitung, die innerhalb von Blutgefäßen an den Behandlungsort geführt wird. Die elektrisch isolierte Leitung ist flexibel, um den Krümmungen und Abzweigungen von Blutgefäßen folgen zu können. Im Hinblick auf das Material der Elektrodenspitze ist lediglich offenbart, dass diese aus Metall gebildet ist.

Die bekannte Elektrode hat den Nachteil, dass die Elektrodenspitze starr oder zumindest nur bedingt flexibel ist. Bei der Verankerung der Elektrodenspitze im Herzmuskel, werden deshalb relativ große Gewebeareale in ihrer Muskelbewegung gehemmt.

Die Aufgabe der Erfindung besteht darin, eine Elektrode zur Messung elektrischer Aktivitäten eines lebenden Körpers und/oder zur Abgabe elektrischer Signale in den lebenden Körper, anzugeben, die eine hohe Flexibilität aufweist und eine zuverlässige Übertragung, insbesondere Senden und Empfangen von elektrischen Signalen ermöglicht. Ferner liegt der Erfindung die Aufgabe zu Grunde, ein Verfahren zum Herstellen einer derartigen Elektrode anzugeben.

Erfindungsgemäß wird diese Aufgabe im Hinblick auf die flexible Elektrode durch den Gegenstand des Patentanspruchs 1 und im Hinblick auf das Verfahren durch den Gegenstand des Patentanspruchs 10 gelöst.

Die Erfindung beruht auf dem Gedanken, eine Elektrode zur Messung elektrischer Aktivitäten eines lebenden Körpers und/oder zur Abgabe elektrischer Signale bzw. Impulse in den lebenden Körper und/oder zur Ablation von Gewebe oder Nervenbahnen oder dem Erregungsleitungssystem im Herz mit einer elektrisch isolierten, flexiblen Leitung anzugeben, die mit einer distal angeordneten, elektrisch leitenden Elektrodenspitze fest oder lösbar verbunden ist, wobei die Elektrodenspitze zur Einfuhr in den Körper angepasst ist. Zumindest eine Tragstruktur der Elektrodenspitze umfasst wenigstens einen kristallinen Formgedächtniswerkstoff. Die Elektrodenspitze ist auf Grund des Formgedächtniswerkstoffes flexibel. Der Formgedächtniswerkstoff der Tragstruktur ist zumindest teilweise durch physikalische Gasphasenabscheidung gebildet derart, dass Ausscheidungen im Formgedächtniswerkstoff eine maximale Größe von 500 nm aufweisen.

Unter einem Formgedächtniswerkstoff wird ein Material verstanden, das die Eigenschaft aufweist, nach einer Formänderung im martensitischen Zustand durch Erwärmen in den Ausgangszustand seine ursprüngliche Form nahezu vollständig oder teilweise anzunehmen. Die Martensitbildung kann auch durch mechanische Beanspruchung bei einer Temperatur oberhalb der Austenit-Finish-Temperatur erfolgen (spannungsinduzierter Martensit)

Der Vorteil des kristallinen Formgedächtniswerkstoffes besteht darin, dass durch den Formgedächtniswerkstoff eine pseudoelastische Formänderung der Tragstruktur der Elektrodenspitze erreichbar ist, die in an sich bekannter Weise durch Bildung von spannungsinduziertem Martensit entsteht. Insofern wird die Flexibilität der Tragstruktur durch den Formgedächtniswerkstoff bewirkt. Die zur Einstellung der pseudoelastischen Eigenschaften des Formgedächtniswerkstoffes erforderlichen Maßnahmen sind dem Fachmann bekannt. Auf Grund der Pseudoelastizität der Tragstruktur der Elektrodenspitze und der damit verbundenen Flexibilität kann sich die gesamte Elektrode, einschließlich der elektrisch isolierten, flexiblen Leitung, die mit der flexiblen Elektrodenspitze fest oder lösbar verbunden ist, gut an unterschiedliche Gefäß- oder Organformen anpassen.

Die Tragstruktur der Elektrodenspitze bildet den Bereich der Elektrodenspitze, der eine tragende oder stützende Funktion erfüllt und zur Aufnahme und Weiterleitung mechanischer Kräfte, bspw. auf Grund von Muskelbewegungen, angepasst ist. Zusätzlich ist die Tragstruktur bzw. der Formgedächtniswerkstoff, insbesondere der metallische Formgedächtniswerkstoff elektrisch leitend. Die Beschichtung einer herkömmlich hergestellten Tragstruktur mit einem anderen Material, bspw. mit Gold, wird nicht als Teil der Tragstruktur selbst angesehen, wenn die Beschichtung nicht zu den mechanischen Eigenschaften der Tragstruktur beiträgt, also keine tragende oder stützende Funktion erfüllt.

Erfindungsgemäß ist ferner vorgesehen, dass der Formgedächtniswerkstoff der Tragstruktur zumindest teilweise durch physikalische Gasphasenabscheidung gebildet ist derart, dass Ausscheidungen im Formgedächtniswerkstoff eine maximale Größe von 500 nm aufweisen. Dadurch unterscheidet sich das Material der Tragstruktur im Hinblick auf das Gefüge von herkömmlich aus Vollmaterial, bspw. durch Umformen konventionell hergestellten Elektrodenspitzen. Die Obergrenze der Größe der Ausscheidungen kann 450 nm, insbesondere 400 nm, insbesondere 350 nm, insbesondere 300 nm, insbesondere 250 nm, insbesondere 200 nm, insbesondere 150 nm, insbesondere 100 nm, insbesondere 75 nm, insbesondere 50 nm, insbesondere 25 nm, insbesondere 20 nm betragen. Die Untergrenze kann 10 nm, insbesondere 15 nm, insbesondere 20 nm, insbesondere 25 nm, insbesondere 30 nm, insbesondere 35 nm, insbesondere 40 nm, insbesondere 45 nm, insbesondere 50 nm betragen. Die vorstehend genannten Untergrenzen können mit den vorstehend genannten jeweils größeren Obergrenzen kombiniert werden.

Die konventionelle Herstellung von Elektrodenspitzen umfasst das Vakuumschmelzen des Ausgangsmaterials, die Warmumformung des Materials bspw. durch Rundhämmern und/oder Warmwalzen sowie die Kaltumformung, z.B. zur Fertigung eines Rohres oder Drahtes in vielen einzelnen Stufen. Häufig erfolgen danach noch weitere Bearbeitungsschritte, z.B. mittels eines Lasers, sowie formgebende Wärmebehandlungen und schließlich das Finishing, z.B. durch Elektropolitur. Im Gegensatz dazu wird bei der Gasphasenabscheidung, insbesondere bei der Sputtertechnologie Targetmaterial erschmolzen und im Vakuum abgegossen. Das so hergestellte Targetmaterial wird zerstäubt und auf einem Substrat abgeschieden (Sputtern). Durch eine nachfolgende Wärmebehandlung wird das amorphe Material in kristallines, superelastisches Material umgewandelt. Alternativ kann die Abscheidung des Materials bei hohen Temperaturen erfolgen derart, dass das Material beim Abscheiden auskristallisiert, bspw. auf einem beheizten Substrat.

Bei der herkömmlichen schmelztechnischen Herstellung des Formgedächtnismaterials werden unvermeidbar Einschlüsse, bspw. TiC und Ti₂NiOₓ gebildet. Diese Einschlüsse entstehen in der Schmelze, wachsen beim Erkalten der Schmelze und liegen dann im erstarrten Material vor. Die schmelztechnisch verursachten Einschlüsse beinhalten i.d.R. Titan, Nickel und dazu Kohlenstoff und/oder Sauerstoff: Die Größe der Einschlüsse beträgt einige µm bis mehr als 20µm. Aufgrund der Schmelzphase kommen derartige Einschlüsse dieser Größe nur bei konventionell hergestelltem Material vor.

Im Rahmen der Anmeldung werden also unter Einschlüssen (inclusions) schmelztechnisch gebildete Phasen verstanden, in denen Fremdatome, wie bspw. Kohlenstoff oder Sauerstoff vorliegen können, die bei der Herstellung und Verarbeitung des Materials von außen eingetragen und somit nicht aus dem Legierungsgefüge ausgeschieden werden.

Durch Dünnschichttechnologie bzw Gasphasenabscheidung, insbesondere Sputtern hergestelltes Material weist hingegen keine Einschlüsse (inclusions) auf, ist also frei von Einschlüssen, weil das Material nicht unmittelbar aus einer Schmelze hergestellt ist, sondern durch Abscheidung des zerstäubten Targetmaterials auf einem Substrat. Dies liegt möglicherweise auch daran, dass die durch Gasphasenabscheidung, insbesondere Sputtern erzielbaren Gehalte an Sauerstoff und Kohlenstoff niedriger sind, als bei herkömmlich auf schmelztechnischem Wege hergestellten Materialien, da die Gasphasenabscheidung, insbesondere das Sputtern im Vakuum erfolgen. Sollte es doch durch Verunreinigungen zu Einschlüssen kommen, sind diese bei Tragstrukturen, die auf der Abscheidungsroute hergestellte sind, wesentlich kleiner als bei Tragstrukturen, die auf der Schmelzroute hergestellt sind. Evtl. Einschlüsse haben eine Größe von weniger als 5 µm, insbesondere weniger als 3 µm, insbesondere weniger als 1 µm, insbesondere weniger als 500 nm, insbesondere weniger als 200 nm, insbesondere weniger als 100 nm, insbesondere weniger als 50 nm, insbesondere weniger als 20 nm, insbesondere weniger als 10 nm. Bei herkömmlich hergestelltem Material kann es zusätzlich zu den Einschlüssen bei der Wärmebehandlung zu Ausscheidungen (precipitates oder precipitations) von Phasen aus der festen Lösung kommen, die durch die mehrfachen Glühbehandlungen und/oder durch die Umformung des Materials wachsen

Die erfindungsgemäß im Formgedächtniswerkstoff der Tragstruktur vorliegenden Ausscheidungen (precipitates oder precipitations) unterscheiden sich auf Grund der Herstellung der Tragstruktur durch Gasphasenabscheidung, insbesondere Sputtern, in Ihrer Größe von den schmelztechnisch bedingten Einschlüssen und sind um einige Größenordnungen kleiner als die schmelztechnisch bedingten Einschlüsse (max. Größe der Ausscheidungen: 500 nm). In der Entstehung unterscheiden sich Ausscheidungen von Einschlüssen darin, dass sich Ausscheidungen als Phase aus der festen Lösung, d.h. aus dem kristallinen Material bilden, also ausgeschieden werden. Einschlüsse bilden sich in der Schmelze durch Keimwachstum und bleiben im erstarrten Material erhalten, bzw. eingeschlossen. Die unterschiedliche Größe der Ausscheidungen / Einschlüsse lässt am Gefüge erkennen, durch welches Verfahren der Formgedächtniswerkstoff der Tragstruktur hergestellt wurde. Das selbe gilt für mögliche Ausscheidungen, die in schmelztechnisch hergestellten Tragstrukturen vorkommen können und größer sind, als Ausscheidungen in Tragstrukturen, die auf der Abscheidungsroute hergestellt sind.

Ausscheidungen bestehen aus den Legierungskomponenten und enthalten i.d.R. keine von außen eingetragenen Fremdatome, da der Herstellungsprozess bei der Gasphasenabscheidung im Vergleich zum konventionellen Prozess sauberer ist. Bspw. bestehen Ausscheidungen bei Formgedächtniswerkstoffen aus NiTi-Legierungen i.d.R. aus Titan und aus Nickel, wobei es verschiedene Ausscheidungstypen mit verschiedenen Zusammensetzungen gibt. Typische auf der Abscheidungsroute erhältliche Ausscheidungen umfassen Ni₄Ti₃.

Ein weiterer Unterschied, der die unterschiedlichen Herstellungsverfahren am Material erkennbar macht, besteht darin, dass die Einschlüssen und Ausscheidungen zeilenförmig in Richtung der Zugrichtung (Rohr oder Draht) bzw. der Walzrichtung (Blech) angeordnet sind, wohingegen die Ausscheidungen des erfindungsgemäßen Formgedächtniswerkstoffes der Tragstruktur isotrop ausgerichtet sind. Die isotrope, also ungerichtete Ausrichtung der Ausscheidungen wirkt sich positiv auf die Leitfähigkeit aus.

Erfindungsgemäß ist die Leitfähigkeit im Wesentlichen homogen in der Elektrodenspitze, insbesondere in der Tragstruktur bzw. homogener als bei herkömmlichen Elektrodenspitzen. Dies liegt daran, dass die Leitfähigkeit neben der Temperaturabhängigkeit u.a. von Einschlüssen beeinflusst wird, an denen die Ladungsträger gestreut werden. Aufgrund der geringen Größe der Ausscheidungen des erfindungsgemäßen Formgedächtniswerkstoffes der Tragstruktur wird die Streuung verringert und die Leitfähigkeit verbessert. Damit ist die erfindungsgemäße Elektrode nicht ausschließlich, aber in besonderer Weise für Anwendungen in der Sensorik geeignet.

Überdies erfüllt die Tragstruktur der Elektrodenspitze eine Doppelfunktion. Auf Grund des pseudoelastischen Effektes des Formgedächtniswerkstoffes weist die Elektrodenspitze einerseits gute mechanische Eigenschaften, insbesondere eine gute Flexibilität auf. Auf Grund der geringen Größe der Ausscheidungen kann mit einer Verbesserung des Ermüdungsverhaltens der Tragstruktur gerechnet werden. Andererseits sind die elektrischen Eigenschaften des Formgedächtniswerkstoffes, insbesondere die Leitfähigkeit, auf Grund der durch die physikalische Gasphasenabscheidung maximalen Größe der Ausscheidungen verbessert.

Zusammengefasst werden bei konventionell hergestellten Elektroden aufgrund der Vielzahl der aufeinanderfolgenden Prozesse (Schmelzen und Erstarren, viele Warm- und Kaltumformungen mit zwischengeschalteten Wärmebehandlungen, insbesondere Zwischenglühungen) undefiniert viele Gitterbaufehler indiziert, die nicht kontrollierbar sind. Diese Gitterfehler wechselwirken untereinander. Z.B. beeinflusst die Dichte der Leerstellen die Versetzungsbildung und die Versetzungsbewegung. Die Kaltverformung bedingt deutlich die Versetzungsbildung und beeinflusst zusammen mit den Glühbehandlungen aber auch stark die Korngröße. Damit lässt sich weder an einen konventionell hergestellten Bauteil genau der elektrische Widerstand vorhersagen bzw. einstellen, noch lassen sich Lot-zu-Lot Unterschiede vermeiden. Bei einer herkömmlichen Elektrode, die aus vielen Einzelkontakten besteht, können überdies die einzelnen Kontakte unterschiedliche elektrische Widerstände haben. Hinzu kommt, dass die finalen Bauteile , insbesondere NiTi-Bauteile, wie die Elektrode oder Elektrodenspitze, welche aus konventionellem Rohr oder Draht hergestellt sind, über den Querschnitt des Bauteils, auch oft über dessen Länge, unterschiedliche elektrische Eigenschaften aufweisen, weil die Gitterfehler über den Querschnitt und/oder die Länge des Bauteils unterschiedlich verteilt sind

Demgegenüber ist eine Elektrodenspitze aus einem Formgedächtnismaterial bzw. deren Tragstruktur, die durch ein PVD-Verfahren, insbesondere durch Sputtern hergestellt ist, reiner, d.h. sie enthält weniger Kohlenstoff und weniger Sauerstoff als konventionelles Material. Die Anzahl der Gitterbaufehler (Leerstellen, Versetzungen) ist geringer als bei konventionellem Material, die sich einschließlich der Korngrenzen aufgrund der geringeren äußeren Beeinträchtigungen (Eintrag von Verunreinigungen) besser kontrollieren lassen. Dies folgt daraus, dass beim Sputtern nur das Ausgangsmaterial erschmolzen wird. Danach erfolgt nur der Sputterprozess und eine einzige Glühbehandlung. Die Schwankungsbreite der Dichte von Leerstellen, Versetzungen oder Korngrenzen über den Querschnitt der Elektrode ist gering. Einschlüsse werden vermieden.

Vorzugsweise weist der Formgedächtniswerkstoff der Tragstruktur eine durch physikalische Gasphasenabscheidung (PVD) gezielt eingestellte maximale Dichte an Materialinhomogenitäten, wie Gitterbaufehler und/oder Grenzflächenfehler und/oder Volumendefekte, auf. Dadurch unterscheidet sich das Material der Tragstruktur weiter von herkömmlich aus Vollmaterial, bspw. durch Umformen hergestellten Elektrodenspitzen, die eine größere Dichte an Materialinhomogenitäten als durch physikalische Gasphasenabscheidung bzw. PVD-Verfahren hergestellte Elektrodenspitzen aufweisen. Fehler im Gefüge des Werkstoffes, d.h. Materialinhomogenitäten, wie Gitterbaufehler (bspw. Leerstellen, Zwischengitteratome, Stufenversetzung, Schraubenversetzung, Stapelfehler) und/oder Grenzflächenfehler und/oder Volumendefekte, behindern ebenfalls den Ladungstransport und verringern die Leitfähigkeit. Durch die auf Grund der physikalische Gasphasenabscheidung Begrenzung der Dichte an Materialinhomogenitäten auf einen maximalen Wert wird die Verringerung der Leitfähigkeit weiter begrenzt und eine insbesondere für die Sensorik vorteilhafte gleichmäßige Leitfähigkeit der Elektrodenspitze erreicht.

Die Materialinhomogenitäten betreffen beispielsweise Versetzungen, Korngrenzen und Einschlüsse im Metallgitter. Diese nulldimensionale und/oder eindimensionalen und/oder zweidimensionalen und/oder dreidimensionalen Gitterbaufehler werden bei der Herstellung der flexiblen Elektrode, insbesondere der Elektrodenspitze kontrolliert.

Vorzugsweise beträgt die mittlere Korngröße des Formgedächtniswerkstoffs der Tragstruktur weniger als 4 µm, insbesondere weniger als 3 µm, insbesondere weniger als 2 µm, insbesondere weniger als 1,5 µm, insbesondere weniger als 1 µm, insbesondere weniger als 0,5 µm, insbesondere weniger als 250 nm, beträgt. Im Unterschied dazu betragen mittlere Korngrößen herkömmlich hergestellter Elektroden 5µm bis 20µm. Überdies kommt es bei abscheidungstechnisch hergestellten Tragstrukturen zu geringeren Schwankungen in der Korngröße als bei konventionellem Material sowohl innerhalb eines Bauteils als auch von Lot zu Lot. Die Korngröße ist homogen, insbesondere homogener als bei schmelztechnisch hergestelltem Material. Durch die homogene mittlere Korngröße wird die Leitfähigkeit weiter verbessert.

Zur Beeinflussung bzw. Einstellung der Größe der Ausscheidungen und der Dichte an Inhomogenitäten, wie linienförmigen Gitterbaufehlern und/oder an Grenzflächenfehlern und/oder an Volumendefekten ist vorteilhaft vorgesehen, die Elektrodenspitze zumindest teilweise durch Sputtern oder durch lasergestützte Abscheidung, herzustellen. Durch die Variation einzelner Prozessparameter des Gasphasenabscheidungsverfahrens können die Inhomogenitäten im Material der Elektrodenspitze gezielt eingestellt werden. Geeignete Prozessvariablen sind beispielsweise der Kammerdruck und/oder der Abscheidedruck und/oder der Partialdruck des Prozessgases und/oder die Targetzusammensetzung.

Durch Variation derartiger Parameter ist in an sich bekannter Weise die Größe der Ausscheidungen bzw. der Gitterbaufehler sowie deren Dichte gezielt einstellbar. Ferner kann auch die Korngröße gezielt eingestellt werden. Durch elektronenmikroskopische Prüfverfahren, insbesondere unter Verwendung eines Rasterelektronenmikroskops bzw. einem Transmissionselektronenmikroskops, können die gezielt eingestellten Inhomogenitäten bzw. Gitterbaufehler kontrolliert werden. Die Qualität und Quantität der gemessenen bzw. erfassten Gitterbaufehler kann zur Einstellung der Prozessparameter des Gasphasenabscheideverfahrens eingesetzt werden.

Die Verwendung von Formgedächtnismaterialien ist in der Medizintechnik an sich bekannt. Die Herstellung von Komponenten aus Formgedächtnismaterialien erfolgt, wie vorstehend erläutert, üblicherweise durch Vakuumschmelzen, Warmumformung, Kaltumformung und abschließende Formgebung. Bei diesem Herstellverfahren treten im Rahmen von Gefügeumwandlungen Volumendefekte, beispielsweise Gitterleerstellen und große Einschlüsse auf, die nicht kontrollierbar sind.

Durch Einsatz des physikalischen Gasphasenabscheideverfahrens werden derartige Gitterfehler reduziert bzw. vermieden. Auf diese Weise wird die Homogenität der elektrischen Leitfähigkeit der Elektrodenspitze und somit die Genauigkeit der Sensorik erhöht. Außerdem wird durch die gezielt eingestellte Dichte von Gitterfehlern in dem Material der Elektrodenspitze eine gleichmäßige Qualität hergestellter Elektroden nicht nur innerhalb eines Produktionsloses bzw. einer Charge, sondern auch zwischen unterschiedlichen Losen bzw. Chargen erreicht. Die Ausschussquote wird somit reduziert.

Vorzugsweise umfasst der Formgedächtniswerkstoff eine Nickel-Titan-Legierung, insbesondere mit einer Zusammensetzung aus 50,8 Atomprozent Nickel und 49,2 Atomprozent Titan. Die Nickel-Titan-Legierung eignet sich besonders, um die erhöhte Flexibilität der Elektrodenspitze, insbesondere die pseudoelastischen Eigenschaften zu erreichen. In der angegebenen, speziellen Zusammensetzung werden ferner Gitterfehler reduziert, so dass die Leitfähigkeit der Elektrodenspitze positiv beeinflussbar ist.

Der Formgedächtniswerkstoff kann eine ternäre oder quaternäre Legierung umfassen. Derartige Legierungen sind einfach und kostengünstig herstellbar.

Bei einer bevorzugten Ausführungsform der erfindungsgemäßen flexiblen Elektrode weist die Elektrodenspitze einen mehrschichtigen Aufbau auf. Durch den mehrschichtigen Aufbau der Elektrodenspitze können mehrere, unterschiedliche elektrische Signale an den lebenden Körper abgegeben bzw. vom lebenden Körper empfangen werden. Die aus mehreren Schichten aufgebaute Elektrodenspitze kann also eine kombinierte Stimulationselektrodenspitze und Ableitungselektrodenspitze bilden. Dabei ist die Stimulationselektrodenspitze zur Energieabgabe in das Körpergewebe und die Ableitungselektrodenspitze zur Ableitung von Signalen aus dem Körpergewebe angepasst. Beispielsweise kann die mehrschichtig aufgebaute Elektrodenspitze einerseits zur Erfassung von elektrischen Aktivitäten von Körperzellen, beispielsweise von Herzmuskelzellen, und gleichzeitig zur Stimulation derselben Körperzellen eingesetzt werden. Andere Applikationen sind möglich, bspw. an Nervenbahnen. Vorzugsweise bestehen eine radial äußere Schicht oder mehrere radial äußere Schichten aus dem Formgedächtniswerkstoff, insbesondere aus einer Nickel-Titan-Legierung, und eine radial innere Schicht oder mehrere radiale innere Schichten aus einem Material mit höherer elektrischer Leitfähigkeit als das Material der radial äußeren Schicht oder radial äußeren Schichten. Insbesondere besteht das Material der radial inneren Schicht oder mehrerer radial innerer Schichten vorzugsweise aus Kupfer, Silber, Gold, Platin, Tantal, Niob, Palladium oder Kohlenstoff. Bei einer zylinderförmigen Elektrodenspitze sind die radial äußere Schicht bzw. die radial äußeren Schichten verstärkt durch Torsion oder Biegung der Elektrodenspitze beansprucht. Die durch den Formgedächtniswerkstoff bereitgestellten pseudoelastischen Eigenschaften kommen in diesen äußeren Schichten besonders vorteilhaft zu tragen, da auf diese Weise die Flexibilität der Elektrodenspitze erhöht wird. Durch die Verwendung von einem Material mit höherer elektrischer Leitfähigkeit in einer oder mehreren radial inneren Schichten wird gewährleistet, dass durch die flexible Elektrodenspitze effizient elektrische Signale in den Körper abgegeben bzw. vom Körper empfangen werden können.

Zwischen den Schichten kann ein in radialer Richtung kontinuierlicher Übergang bestehen. Die Elektrodenspitze kann also im Wesentlichen dem Aufbau einer Gradientenfaser entsprechen, wobei sich zwischen dem radialen Außenumfang der Elektrodenspitze und der Längsachse der Elektrodenspitze ein Materialgradient einstellt. Das Material der äußeren Schichten und das Material der inneren Schichten können ineinander verlaufen bzw. fließend ineinander übergehen.

Die radial innere Schicht oder die radial inneren Schichten können aus einer Nickel-Titan-Legierung und die radial äußere Schicht oder die radial äußeren Schichten können aus einem Material mit höherer elektrischer Leitfähigkeit als das Material der radial inneren Schicht oder der radial inneren Schichten hergestellt sein. Ein derartiger Schichtaufbau ist vorteilhaft, wenn über die Länge der Elektrodenspitze, insbesondere über den Außenumfang der zylindrischen Elektrodenspitze, elektrische Signale übertragen werden sollen.

Gemäß einem nebengeordneten Aspekt beruht die Erfindung auf dem Gedanken, ein Verfahren zur Herstellung einer Elektrode zur Messung elektrischer Aktivitäten eines lebenden Körpers und/oder zur Abgabe elektrischer Signale in den lebenden Körper anzugeben. Die Elektrode weist eine elektrisch isolierte, flexible Leitung auf, die mit einer distal angeordneten, elektrisch leitenden Elektrodenspitze fest oder lösbar verbunden ist, wobei die Elektrodenspitze zur Einfuhr in den Körper angepasst ist. Bei dem Verfahren wird zumindest eine Tragstruktur der Elektrodenspitze aus wenigstens einem kristallinen Formgedächtniswerkstoff zumindest teilweise durch physikalische Gasphasenabscheidung gebildet derart, dass die Elektrodenspitze auf Grund des Formgedächtniswerkstoffes flexibel ist

Die physikalische Gasphasendampfabscheidung kann bspw. in Form von Sputtern oder als lasergestützte Abscheidung erfolgen. Dabei ist das Sputtern, bzw. die Kathodenzerstäubung, insbesondere das Magnetronsputtern als besonders geeignetes Verfahren hervorzuheben. Derartige PVD-Verfahren sind dem Fachmann an sich bekannt.

Das erfindungsgemäße Verfahren hat den Vorteil, dass die maximale Dichte von Materialinhomogenitäten, wie Gitterfehlern in der Elektrodenspitze gezielt eingestellt werden kann, beispielsweise durch Variation geeigneter Prozessparameter, wie Kammerdruck und/oder Abscheidedruck und/oder Partialdruck des Prozessgases und/oder Targetzusammensetzung. Somit kann auf besonders einfache Weise eine flexible Elektrodenspitze bereitgestellt werden, die verbesserte Eigenschaften hinsichtlich der Leitfähigkeit aufweist. Das erfindungsgemäße Herstellverfahren gewährleistet ferner eine gleichmäßige Qualität der hergestellten Elektroden nicht nur innerhalb einer Charge, sondern auch über mehrere Chargen hinweg. Insbesondere kann mit dem erfindungsgemäßen Herstellverfahren die Ausschussquote reduziert werden.

In einer besonderen Ausgestaltung des erfindungsgemäßen Verfahrens ist vorgesehen, mehrere Schichten aus unterschiedlichen Materialien zur Bildung der Elektrodenspitze aufzubringen, wobei die radial inneren Schichten und die radial äußeren Schichten durch eine Kombination aus einem Lithografieverfahren und einem Sputterverfahren hergestellt werden. Mit Hilfe des Lithographieverfahrens können die inneren bzw. äußeren Schichten der Elektrodenspitze strukturiert werden, so dass besondere Oberflächenstrukturen oder geometrische Anordnungen der unterschiedlichen Materialien erreicht werden. Durch die Kombination des Lithographieverfahrens mit dem Sputterverfahren können auf einfache Weise unterschiedliche Geometrien bzw. Anordnungen des Schichtaufbaus der Elektrodenspitze hergestellt werden. Aufgrund der kurzen Umrüstzeiten bei Verwendung des Lithographieverfahrens und des Sputterverfahrens können kurzfristig unterschiedliche Elektroden hergestellt werden, die jeweils eine unterschiedliche geometrische Anordnung der verschiedenen Materialien umfassen.

Es ist auch möglich, mit dem Verfahren Leiterbahnen und Schaltkreise in die Elektrodenspitze durch Sputtern zu integrieren. Insofern werden sowohl ein entsprechendes Herstellungsverfahren als auch eine entsprechende Elektrodenspitze, bzw. eine Elektrode mit einer entsprechenden Elektrodenspitze offenbart.

Die im Zusammenhang mit der eingangs beschriebenen Elektrode genannten Vorteile und Wirkungen gelten auch für das Herstellverfahren und werden in diesem Zusammenhang ebenfalls offenbart. Dasselbe gilt für die im Zusammenhang mit der Elektrode beschriebenen Prozessschritte.

Die Erfindung wird im Folgenden anhand von Ausführungsbeispielen, unter Bezugnahme auf die beigefügten, schematischen Zeichnungen, näher erläutert. Darin zeigen
- Fig. 1: einen Querschnitt durch eine Elektrodenspitze einer erfindungsgemäßen Elektrode gemäß einem bevorzugten Ausführungsbeispiel;
- Fig. 2: einen Querschnitt durch eine Elektrodenspitze einer erfindungsgemäßen Elektrode gemäß einem weiteren bevorzugten Ausführungsbeispiel;
- Fig. 3a: einen Querschnitt durch eine Elektrodenspitze einer erfindungsgemäßen Elektrode gemäß einem weiteren bevorzugten Ausführungsbeispiel;
- Fig. 3b: eine Draufsicht auf die Elektrodenspitze gemäß Fig. 3a;
- Fig. 4: eine Draufsicht auf eine Elektrodenspitze einer erfindungsgemäßen Elektrode gemäß einem weiteren bevorzugten Ausführungsbeispiel;
- Fig. 5: einen Querschnitt durch eine Elektrodenspitze einer erfindungsgemäßen Elektrode gemäß einem weiteren bevorzugten Ausführungsbeispiel;
- Fig. 6a: einen Querschnitt durch eine Elektrodenspitze einer erfindungsgemäßen Elektrode gemäß einem weiteren Ausführungsbeispiel;
- Fig. 6b: eine Seitenansicht der Elektrodenspitze gemäß Fig. 6a;
- Fig. 7: ein Schliffbild (500-fache Vergrößerung) einer erfindungsgemäß hergestellten Elektrodenspitze und
- Fig. 8: ein Schliffbild (500-fache Vergrößerung) einer herkömmlich hergestellten Elektrodenspitze.

Den nachfolgend beschriebenen Ausführungsbeispielen liegt im Wesentlichen dasselbe Herstellungsverfahren zu Grunde, wobei die Elektrodenspitze, insbesondere die Tragstruktur der Elektrodenspitze durch ein Gasphasenabscheideverfahren, insbesondere ein Sputterverfahren aus einem Formgedächtniswerkstoff hergestellt wird. Die Tragstruktur kann einen Hohlkern zur Bildung einer Hohlelektrode umfassen. Alternativ kann die Tragstruktur aus Vollmaterial, d.h. ohne Hohlkern zur Bildung einer massiven Elektrodenspitze ausgebildet sein.

Die gesamte Tragstruktur kann durch das Gasphasenabscheideverfahren hergestellt sein. Es ist auch möglich, einen Tragstrukturverbund aus unterschiedlichen Materialien und/oder aus unterschiedlich hergestellten Materialien zu bilden. So kann ein Teil der Tragstruktur, bspw. ein äußerer Bereich der Tragstruktur durch Sputtern und ein weiterer Teil der Tragstruktur, bspw. ein Kern durch ein herkömmliches Verfahren, bspw. durch Umformen hergestellt sein. Es ist auch möglich, den inneren Bereich durch Sputtern und den äußeren Bereich durch ein herkömmliches Verfahren, bspw. durch Umformen herzustellen und die Bereiche daraufhin zu fügen. Durch eine geeignete Wahl der Materialien, bspw. Formgedächtniswerkstoffe für beide Bereiche kann die Flexibilität des Tragstrukturverbundes erreicht werden. Es ist auch möglich mehr als zwei verschiedene Bereiche der Tragstruktur unterschiedlich herzustellen und/oder unterschiedliche Materialien zu verwenden.

Es ist möglich 30%-90% des Materials der Tragstruktur durch das Gasphasenabscheideverfahren herzustellen. Die Untergrenze des Bereichs kann 35%, insbesondere 40%, insbesondere 45%, insbesondere 50%, insbesondere 55%, insbesondere 60% betragen. Die Obergrenze des Bereichs kann 85%, insbesondere 80%, insbesondere 75%, insbesondere 70%, insbesondere 65% betragen. Die vorstehend genannten Bereichsgrenzen können miteinander kombiniert werden.

Die Tragstruktur oder der gesputterte Bereich der Tragstruktur weist eine Dicke, insbesondere in radialer Richtung bzw. eine Wandstärke von mindestens 5 µm, insbesondere mindestens 10 µm, insbesondere mindestens 15 µm, insbesondere mindestens 20 µm, insbesondere mindestens 25 µm, insbesondere mindestens 30 µm, insbesondere mindestens 35 µm, insbesondere mindestens 40 µm, insbesondere mindestens 45 µm, insbesondere mindestens 50 µm, insbesondere mindestens 100 µm, insbesondere mindestens 200 µm, insbesondere mindestens 300 µm, insbesondere mindestens 400 µm, insbesondere mindestens 500 µm auf. Das gilt für jeden einzelnen Bereich, der gesputtert wird, oder auch für die Summe der Dicke aller gesputterten Bereiche (bspw., wenn mehrere Materialien eingesetzt werden) Die Obergrenze der Wandstärke kann bspw. höchstens 5mm, insbesondere höchstens 4,5mm, insbesondere höchstens 4mm, insbesondere höchstens 3,5mm, insbesondere höchstens 3mm, insbesondere höchstens 2,5mm, insbesondere höchstens 2mm, insbesondere höchstens 1,5mm, insbesondere höchstens 1mm, insbesondere höchstens 0,5mm betragen. Die vorstehend genannten Obergrenzen werden im Zusammenhang mit den vorstehend genannten Untergrenzen offenbart.

Die Stabilität der Tragstruktur bei geringen Wandstärken, wie bspw. bei Folien mit einer Dicke von mindestens 5 µm, insbesondere von mindestens 10 µm, insbesondere von mindestens 15 µm, insbesondere von mindestens 20 µm wird durch die Geometrie der Elektrodenspitze erreicht. Bspw. ist eine hohlzylindrisch geformte Folie ausreichend stabil, um als Tragstruktur verwendet zu werden. Die Länge und den Durchmesser der hohlzylindrischen Tragstruktur bestimmt der Fachmann derart, dass sich eine ausreichend stabile Elektrodenspitze ergibt.

Das Sputterverfahren kann auch für weitere Werkstoffe, die Bestandteil der Elektrodenspitze sein können, eingesetzt werden. Beim Sputtern bzw. allgemein bei der Ionenstrahlassistierten Abscheidung ist eine kombinierte thermische Elektronenstrahlbedampfung mit gleichzeitigem Ionenbeschuss auf ein Substrat vorteilhaft, wobei ein inertes Gas, beispielsweise Argon, Xenon, Stickstoff oder Neon eingesetzt wird. Durch den Beschuss mit inertem Gas, beispielsweise Argonionen, kann die Gitterfehlerdichte im Material der Elektrodenspitze reduziert werden. Alternative Abscheideverfahren, die zur Herstellung der Elektrodenspitze 10 bzw. allgemein der Elektrode einsetzbar sind, umfassen Laserablation und direkte Ionenstrahlabscheidung, sowie Abscheideverfahren basierend auf kathodischen Lichtbogenentladungen. Vakuumabscheideverfahren eignen sich besonders zur Einstellung der mechanischen Eigenschaften der Elektrodenspitze 10 durch die Werkstoffeigenschaften des verwendeten Materials, insbesondere die Einstellung der Gitterfehlerdichte. Durch eine geeignete, dem Abscheideverfahren nachgeordnete, Behandlung, beispielsweise Ausglühen bzw. Auslagern, Hochdruckbehandlung oder Abschrecken, insbesondere mittels Gas, können die mechanischen Eigenschaften verbessert bzw. direkt die Gitterfehlerdichte weiter verringert werden. Vorteilhafterweise ist eine geeignete Steuereinrichtung vorgesehen, die die Prozessparameter kontrolliert und das Herstellverfahren entsprechend beeinflusst. Insbesondere ist die Steuereinrichtung angepasst, um die folgenden Prozessschritte zu überwachen und zu steuern bzw. zu regeln.

Zur Beeinflussung der Gitterfehlerdichte des Materials der Elektrodenspitze 10 können unterschiedliche Prozessparameter variiert werden. Insbesondere ist die Gitterfehlerdichte vom Kammerdruck, dem Abscheidedruck und dem Partialdruck des Prozessgases während der Herstellung bzw. der Abscheidung des Materials abhängig. Während des Herstellungsverfahrens werden daher derartige Parameter kontinuierlich erfasst und mit zuvor bestimmten Sollwerten verglichen, um die Qualität der hergestellten Elektrodenspitze 10 kontinuierlich sicherzustellen. Insbesondere die reaktiven und nichtreaktiven Gase, die bei den Abscheideverfahren zum Einsatz kommen, werden kontrolliert bzw. geeignet dosiert. Die verwendeten inerten oder nichtreaktiven Gase umfassen vorzugsweise Argon bzw. Stickstoff. Das Substrat, auf das die oder mehrere Schichten von Materialien für die Elektrodenspitze 10 abgeschieden werden, kann feststehend oder beweglich sein. Beispielsweise kann das Substrat um eine Längsachse rotiert werden, um eine dreidimensionale, gesputterte Elektrodenspitze 10 herzustellen. Ein bewegliches Substrat eignet sich insbesondere zur Herstellung von zylinderförmigen Elektrodenspitzen 10 bzw. allgemein für Elektrodenspitzen 10, die eine gekrümmte, insbesondere rotationssymmetrische, Oberfläche aufweisen. An Stelle oder zusätzlich zu einer Rotation des Substrats können weitere Bewegungen des Substrats innerhalb eines Koordinatensystems vorgesehen sein, um besondere Geometrien für die Elektrodenspitze 10 darzustellen.

Im Allgemeinen kann das Material der Elektrodenspitze 10 zur Bildung der Tragstruktur gleichförmig auf dem Substrat abgeschieden werden. Alternativ kann das Material derart auf dem Substrat abgeschieden werden, dass sich eine Strukturierung ergibt. Die Strukturierung kann durch ein Lithographieverfahren, insbesondere ein Photolithographieverfahren, vorgegeben werden.

Das auf dem Substrat abgeschiedene Material kann auf unterschiedliche Art und Weise von dem Substrat gelöst werden. Beispielsweise kann das abgeschiedene Material durch chemisches Ätzen oder chemisches Ablösen oder mechanisch von dem Substrat entfernt werden. Es ist auch möglich, zwischen dem Substrat und dem Material für die Elektrodenspitze 10 eine Opferschicht vorzusehen, die zum Ablösen der hergestellten Elektrodenspitze 10 vom Substrat durch chemisches Ätzen oder Schmelzen, oder andere geeignete Verfahren entfernt wird.

Damit wird eine Hohlelektrode gebildet, deren tragende Struktur bzw. Tragstruktur durch das PVD-Verfahren, insbesondere durch das Sputtern hergestellt ist. Die tragende Struktur bedingt die mechanischen und elektrischen Eigenschaften der Hohlelektrode bzw. Hohlelektrodenspitze. Die Wandstärke der Hohlelektrodenspitze kann gering sein und bspw. mindestens 5 µm, insbesondere mindestens 10 µm, insbesondere mindestens 15 µm, insbesondere mindestens 20 µm, insbesondere mindestens 25 µm, insbesondere mindestens 30 µm, insbesondere mindestens 35 µm, insbesondere mindestens 40 µm, insbesondere mindestens 45 µm, insbesondere mindestens 50 µm, insbesondere mindestens 100 µm, insbesondere mindestens 200 µm, insbesondere mindestens 300 µm, insbesondere mindestens 400 µm, insbesondere mindestens 500 µm umfassen. Die Hohlelektrodenspitze kann auf einem Führungsdraht geführt sein. Es ist auch möglich, einen Kern in der Elektrodenspitze vorzusehen, der von einer gesputterten Schicht aus einem Formgedächtniswerkstoff umgeben ist, wobei die Schicht so dick ist, dass diese zusammen mit dem Kern eine tragende Funktion übernimmt.

Die Größe der beim Abscheiden des Targetmaterials gebildeten Ausscheidungen ist begrenzt. Konkret beträgt die Obergrenze 500nm. Niedrigere Obergrenzen sind möglich. So kann die Obergrenze der Größe der Ausscheidungen 450 nm, insbesondere 400 nm, insbesondere 350 nm, insbesondere 300 nm, insbesondere 250 nm, insbesondere 200 nm, insbesondere 150 nm, insbesondere 100 nm, insbesondere 75 nm, insbesondere 50 nm, insbesondere 25 nm, insbesondere 20 nm betragen. Die Untergrenze kann 10 nm, insbesondere 15 nm, insbesondere 20 nm, insbesondere 25 nm, insbesondere 30 nm, insbesondere 35 nm, insbesondere 40 nm, insbesondere 45 nm, insbesondere 50 nm betragen und mit den vorstehend genannten jeweils größeren Obergrenzen kombiniert werden. Mit abnehmender Größe der Ausscheidungen wird die Leitfähigkeit des Materials weiter verbessert, da die Ladungsträger weniger stark an den Ausscheidungen gestreut werden.

Die gezielte Einstellung der Größe und/oder Dichte von Gitterbaufehlern durch die Gasphasenabscheidung, insbesondere das Sputtern, ist eine weitere Maßnahme, wie die Leitfähigkeit verbessert werden kann. Dies betrifft nulldimensionale und/oder eindimensionale und/oder zweidimensionale und/oder dreidimensionale Gitterfehler. Nulldimensionale Gitterfehler sind bspw. Leestellen, Zwischengitteratome und Fremdatome. Eindimensionale Gitterfehler umfassen Versetzungen, insbesondere Stufenversetzungen und Schraubenversetzungen. Zweidimensionale Gitterfehler können Korngrenzen, Stapelfehler und Zwillingsgrenzen sein. Dreidimensionale Gitterfehler sind Poren, Einschlüsse und Ausscheidungen. Da bei Formgedächtniswerkstoffen Ausscheidungen für die Eigenschaften der Formgedächtniswerkstoffe maßgeblich sind, ist die Begrenzung der Größe der Ausscheidungen eine besonders wirksame Maßnahme. Bei den übrigen Gitterbaufehlern wird deren Dichte und/oder sofern sinnvoll deren Größe gezielt eingestellt. Zusätzlich zur Begrenzung der Größe der Ausscheidungen kann deren Dichte gezielt eingestellt werden.

Die Elektrodenspitze kann aus einer einzigen Schicht (nicht dargestellt) oder aus wenigstens zwei Schichten 11, 12 aufgebaut sein. Dies gilt insbesondere, wenn die Elektrodenspitze eine im Wesentlichen zylinderförmige, insbesondere hohlzylindrische bzw. rotationssymmetrische Form aufweist. Alternativ kann die Elektrodenspitze 10 auch eine planare Struktur, beispielsweise eine Platte bzw. ein Plättchen, bilden. Im Falle einer planaren Struktur kann die Elektrodenspitze 10 wenigstens zwei Bereiche 11, 12 mit verschiedenen Materialien umfassen.

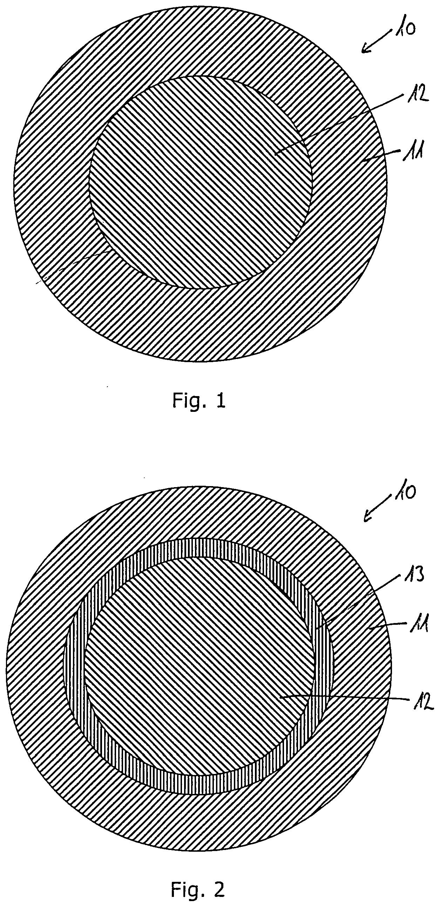

Eine mögliche Grundstruktur einer im Wesentlichen zylinderförmigen Elektrodenspitze 10 mit zwei unterschiedlichen Materialschichten 11, 12 ist beispielhaft in Fig. 1 dargestellt. Die Tragstruktur ist dabei aus Vollmaterial gebildet. Die Grundstruktur kann auf mehr als zwei Schichten erweitert werden.

In Fig. 1 ist beispielhaft der Aufbau einer Elektrodenspitze 10 gezeigt, die eine rotationssymmetrische Form aufweist und aus zwei Schichten 11, 12 gebildet ist. Die innere Schicht 12 bzw. die Kernschicht umfasst vorzugsweise ein Material, das eine höhere Leitfähigkeit aufweist als die äußere Schicht 11 bzw. die Mantelschicht. Beispielsweise kann die innere Schicht 12 Kupfer, Silber, Gold, Platin, Tantal, Niob, Palladium oder Kohlenstoff umfassen. Die innere Schicht 12 kann beispielsweise bei der Herstellung der Elektrodenspitze als Draht bereitgestellt werden. Der Draht kann während des Herstellvorgangs als Substrat für die äußere Schicht 11 bzw. die Mantelschicht dienen. Die Mantelschicht bzw. die äußere Schicht 11 kann durch einen Sputterprozess auf der inneren Schicht 12 abgeschieden werden. Die äußere Schicht 11 und die innere Schicht 12 können fließend bzw. kontinuierlich ineinander übergehen. Das bedeutet, dass zwischen den äußeren und inneren Schichten 11, 12 keine klar abgegrenzten Schichtgrenzen bestehen. Vielmehr kann sich ein radialer Materialgradient in der Elektrodenspitze 10 einstellen. Der Materialgradient kann bspw. durch Ansteuern mehrerer Targets mit unterschiedlichen Zusammensetzungen eingestellt werden, so dass sich die Materialzusammensetzung der Elektrodenspitze 10 kontinuierlich ändert.

Die innere Schicht 12 kann ferner ein Material umfassen, das eine erhöhte Röntgensichtbarkeit gewährleistet. Auf diese Weise kann die Position der Elektrodenspitze 10 innerhalb des lebenden Körpers durch ein bildgebendes Verfahren, insbesondere basierend auf Röntgenstrahlung, kontrolliert werden.

Die äußere Schicht 11 umfasst vorzugsweise einen Formgedächtniswerkstoff. Der Formgedächtniswerkstoff weist eine kristalline Struktur auf, wobei die Inhomogenitäten in der Kristallstruktur gezielt eingestellt sind, insbesondere durch Variation der Parameter des Sputterprozesses während der Herstellung. Die hierfür erforderlichen Maßnahmen bestimmt der Fachmann bspw. experimentell. Vorzugsweise umfasst die äußere Schicht 11 eine Nickel-Titan-Legierung, insbesondere Nitinol. Im Allgemeinen weist die äußere Schicht 12 mechanische Eigenschaften in Kombination mit elektrisch leitenden Eigenschaften auf. Die innere Schicht 12 weist hingegen vorwiegend leitende Eigenschaften in Kombination mit Röntgensichtbarkeitseigenschaften auf. Die vorgenannten Merkmale gelten für alle Ausführungsbeispiele.

Ein weiteres Ausführungsbeispiel der Tragstruktur einer Elektrodenspitze zeigt Fig. 2, wobei zwischen der äußeren Schicht 11 und der inneren Schicht 12 eine Zwischenschicht 13 angeordnet ist. Die Zwischenschicht 13 kann beispielsweise isolierende Eigenschaften aufweisen. Insbesondere kann die Zwischenschicht 13 ein isolierendes Kunststoffmaterial umfassen. Auf diese Weise können unterschiedliche Signale bzw. elektrische Ströme über die Elektrodenspitze 10 transportiert werden. Beispielsweise kann mit Hilfe der isolierenden Zwischenschicht 13 eine bipolare Elektrodenspitze 10 bereitgestellt werden. Es ist auch möglich, dass eine der Schichten 11, 12 beispielsweise die äußere Schicht zur Ableitung von elektrischen Signalen aus dem Körpergewebe, beispielsweise zur Messung der elektrischen Aktivität des Körpergewebes, eingesetzt wird, wogegen die innere Schicht zur Zufuhr eines elektrischen Stroms in das Körpergewebe, beispielsweise zum Zwecke der Stimulation des Körpergewebes, genutzt wird.

Konkret können mit der äußeren Schicht 11 beispielsweise elektrische Signale des Herzmuskels, insbesondere der Schrittmacherzellen, erfasst werden. Über eine geeignete Messeinrichtung können die gemessenen elektrischen Signale ausgewertet werden. Die innere Schicht 12 dient zur Einleitung eines Stromimpulses in die Herzmuskelzellen, wobei der Stromimpuls in Abhängigkeit des Messergebnisses der über die äußere Schicht 11 übertragenen Signale erzeugt wird. Ein derartiger Aufbau der Elektrodenspitze 10 ist beispielsweise bei Elektroden für Herzschrittmacher bzw. Herzschrittmacher-/Defibrillator-Vorrichtungen vorteilhaft. Es ist nicht ausgeschlossen, dass die Materialien und/oder Funktionen der Schichten 11, 12 vertauscht sind. Beispielsweise kann die äußere Schicht 11 ein Material mit einer erhöhten elektrischen Leitfähigkeit, beispielsweise zur Stimulation von Körperzellen, aufweisen und die innere Schicht 12 einen Formgedächtniswerkstoff umfassen, der vorwiegend mechanische Eigenschaften mit elektrisch leitenden Eigenschaften kombiniert, wobei die innere Schicht 12 zur Erfassung von elektrischen Signalen der Körperzellen eingesetzt wird.

Vorteilhafterweise werden wenigstens die äußere Schicht 11 und/oder die Zwischenschicht 13 und/oder die innere Schicht 12, durch ein Sputterverfahren hergestellt. Damit wird eine relativ kleine Wandstärke erreicht, wobei die mechanischen Eigenschaften, insbesondere die Flexibilität der Elektrodenspitze 10, aufgrund der pseudoelastischen Eigenschaften des Formgedächtniswerkstoffs erhöht sind. Dies gilt insbesondere für die isolierende Zwischenschicht 13, die eine vergleichsweise dünne Wandstärke aufweisen kann, ohne dass die isolierenden Eigenschaften beeinträchtigt werden. Insbesondere kann durch die relativ homogene Struktur der gesputterten Zwischenschicht 13 erreicht werden, dass die isolierenden Eigenschaften auch dann beibehalten werden, wenn die flexible Elektrodenspitze 10 einer starken Biegebeanspruchung unterliegt.

Hervorzuheben ist allgemein die Doppelfunktion des Formgedächtniswerkstoffes als elektrischer Leiter einerseits und als tragendes Material andererseits, das die mechanischen Eigenschaften der Elektrodenspitze 10 bestimmt. Dies bedeutet, dass die Stärke des Materialbereichs, der aus dem Formgedächtniswerkstoff gebildet ist, derart dimensioniert ist, dass dieser eine tragende Funktion übernimmt. Dies ist bspw. der fall bei einer Hohlelektrode, deren Wandung aus dem Formgedächtniswerkstoff gebildet ist.

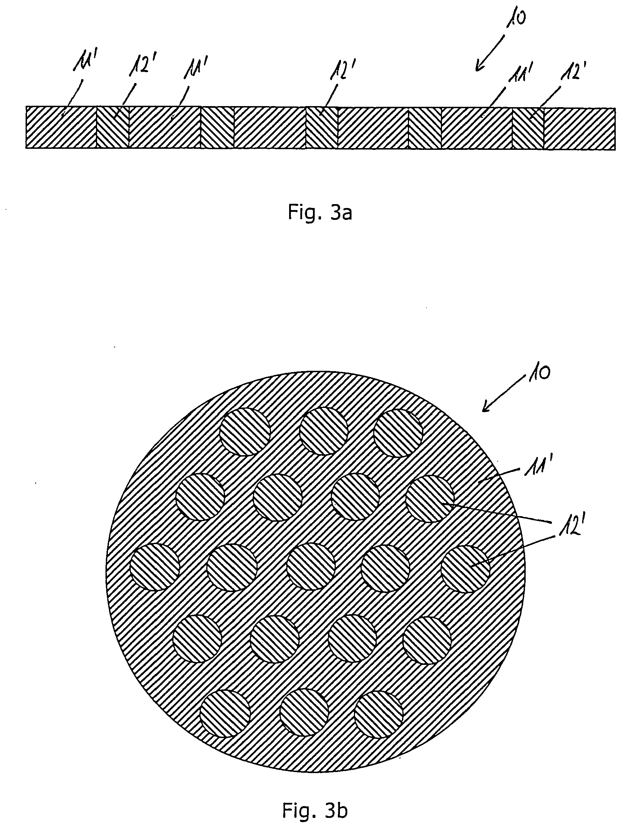

Alternativ zu einer zylinderförmigen Struktur der flexiblen Elektrodenspitze 10 kann die Elektrodenspitze 10 eine planare bzw. flache Form aufweisen, wie in den Fig. 3a und 3b dargestellt. Es ist möglich, die planare bzw. flache Form als eine einzige Schicht (nicht dargestellt) zu realisieren. Die planare Struktur besteht aus einem gesputterten Vollmaterial. Im Fall zweier Schichten sind anstelle der äußeren und inneren Schicht 11, 12 äußere und innere Bereiche 11', 12' vorgesehen, die im Wesentlichen dieselben Eigenschaften wie die äußeren und inneren Schichten 11, 12 gemäß den vorbeschriebenen Ausführungsbeispielen aufweisen. Die Grundstruktur mit zwei bereichen kann auf andere Strukturen mit mehr als zwei Bereichen übertragen werden. In der Querschnittsdarstellung gemäß Fig. 3a ist zu erkennen, dass sich die äußeren und inneren Bereiche 11', 12' abwechseln können. Dabei können die inneren Bereiche 12' in eine Grundstruktur des äußeren Bereichs 11' eingebettet sein, wie aus Fig. 3b ersichtlich. Die Grundstruktur des äußeren Bereichs 11' bildet die Tragstruktur. Vorzugsweise sind die inneren Bereiche 12' durch ein elektrisch besonders leitfähiges Material gekennzeichnet, wogegen das Grundmaterial des äußeren Bereichs 11' einen Formgedächtniswerkstoff umfasst, der die erhöhte Flexibilität der Elektrodenspitze 10 maßgeblich beeinflusst. Insgesamt kann die flexible Elektrodenspitze 10 gemäß Fig. 3b eine kreisrunde, plattenartige Struktur aufweisen, wobei der Grundkörper der flexiblen Elektrodenspitze 10 durch den äußeren Bereiche 11', umfassend den Formgedächtniswerkstoff, gebildet ist. In dem äußeren Bereich 11' sind mehrere innere Bereiche 12 eingebettet, die ein unterschiedliches Material, insbesondere ein Material mit einer elektrischen Leitfähigkeit aufweisen, die die Leitfähigkeit des Materials des äußeren Bereichs 11' übersteigt.

Die plattenartige Elektrodenspitze 10 kann auf unterschiedliche Weise strukturiert sein. Bei dem Ausführungsbeispiel gemäß Fig. 3a und 3b weist die plattenartige flexible Elektrodenspitze 10 eine im Wesentlichen gepunktete Struktur auf. Es ist auch möglich, dass die Elektrodenspitze 10 eine im Wesentlichen leiterplattenähnliche Struktur, beispielsweise nach Art einer Elektronikplatine, umfasst. Andere Strukturierungen sind möglich. Insbesondere kann die Elektrodenspitze 10 eine dreidimensionale Strukturierung aufweisen, wobei beispielsweise die inneren Bereiche 12' über den äußeren Bereich 11' vorstehen können.

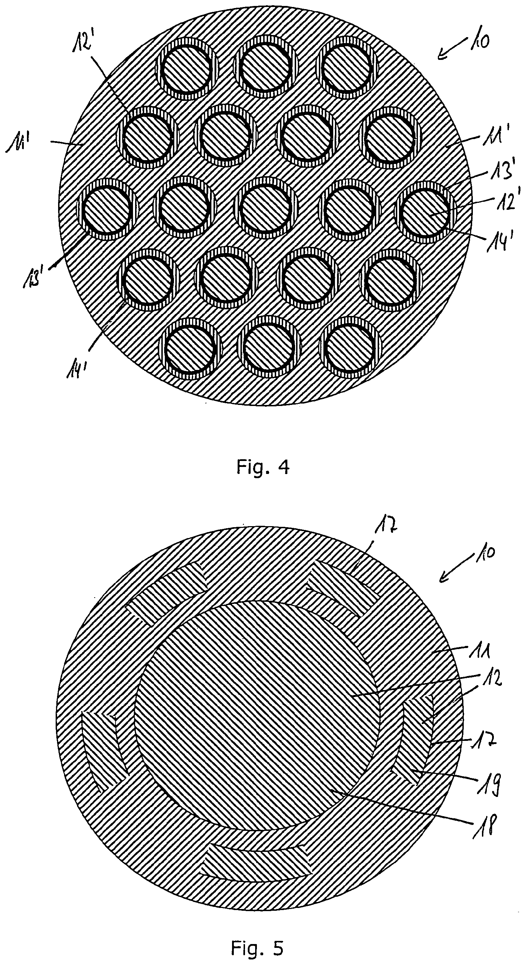

Wie in Fig. 4 dargestellt, kann die plattenförmige Elektrodenspitze 10 weitere Bereiche, insbesondere einen Zwischenbereich 13' und einen Trennbereich 14' umfassen. Der Trennbereich 14' und der Zwischenbereich 13' können konzentrisch den inneren Bereich 12' umgebend angeordnet sein. Der Zwischenbereich 13' weist in Analogie zur Zwischenschicht 13 bei der zylinderförmigen Elektrodenspitze 10 gemäß Fig. 2 ein elektrisch isolierendes Material auf. Der Zwischenbereich 13' umgibt ringförmig den inneren Bereich 12' und den Trennbereich 14'. Der Zwischenbereich 13' isoliert also den Trennbereich 14' vom äußeren Bereich 11'. Der Trennbereich 14' kann ebenfalls ein isolierendes oder ein elektrisch leitendes Material umfassen. Vorzugsweise weist der Trennbereich 14' ein elektrisch leitendes Material auf.

Es ist vorteilhaft, die planare bzw. flache Elektrodenspitze 10 durch einen Sputterprozess herzustellen. Die planare Struktur des äußeren Bereichs 11' kann nach dem Sputtern strukturiert werden, um die einzelnen Bereiche 11', 12', 13', 14' voneinander zu trennen. Die Strukturierung kann beispielsweise durch einen lithografischen Prozess erfolgen. Die planare Struktur der Elektrodenspitze 10, insbesondere des äußeren Bereichs 11' kann nasschemisch geätzt werden. Zwischen den einzelnen Bereichen 11', 12', 13', 14' können im Wesentlichen zur Oberfläche der Elektrodenspitze 10 senkrecht angeordnete Bereichsgrenzen ausgebildet sein. Bei der Strukturierung der Elektrodenspitze 10 durch ein nasschemisches Ätzverfahren können auch schräg angeordnete Bereichsgrenzen gebildet werden. Die planare bzw. flache Elektrodenspitze 10 kann vollständig mit einem Oxid überzogen bzw. besputtert werden. Alternativ oder zusätzlich kann die Elektrodenspitze 10 eine Kunststoffschicht aufweisen, auf die die einzelnen Bereiche 11', 12', 13', 14' abgeschieden werden. Die Kunststoffschicht bzw. der äußere Bereich 11' kann eine Sputterbasis bilden, die während des Sputterprozesses als Substrat dient. Durch einen galvanischen Prozess kann das isolierende Material der Basis in den Zwischenräumen zwischen den einzelnen elektrisch leitenden Bereichen 12', 13', 14' wachsen. Im Allgemeinen können mehrere unterschiedliche Materialien vorgesehen sein, die entweder durch zusätzliche, dazwischen angeordnete Bereiche, voneinander isoliert sind oder miteinander einen elektrischen Kontakt bilden.

Es ist auch möglich, die Platte aus einem einzigen Material, insbesondere aus einem einzigen Formgedächtniswerkstoff durch ein PVD-Verfahren herzustellen.

Die zylindrische Form bzw. generell die dreidimensionale Form der Elektrodenspitze kann beispielsweise durch eine Wärmebehandlung eingestellt werden, so dass sich die Form der Elektrode durch den Formgedächtniseffekt ergibt. Alternativ kann die dreidimensionale Form der Elektrode durch Umformen der planaren Elektrodenspitze, insbesondere durch Rollen der Elektrodenspitze hergestellt werden. Die flache Elektrodenspitze 10 bildet in diesem Fall das Vorprodukt bzw. ein Zwischenprodukt vor der endgültigen Herstellung der zylindrischen bzw. dreidimensionalen Elektrodenspitze.

Bei dem Ausführungsbeispiel gemäß Fig. 4 ist vorteilhaft vorgesehen, dass der äußere Bereich 11' und der Trennbereich 14' im Wesentlichen isolierende Eigenschaften aufweisen. Der innere Bereich 12' und der Zwischenbereich 13' umfassen jeweils elektrisch leitende Materialien. Die elektrisch leitenden Bereiche 12', 13' sind auf bzw. in der Struktur des äußeren Bereichs 11', der im Wesentlichen isolierend wirkt, zueinander konzentrisch angeordnet. Zwischen dem inneren Bereich 12' und dem Zwischenbereich 13' ist ein konzentrisch angeordneter Trennbereich 14' vorgesehen, der ein isolierendes Material umfasst und den elektrisch leitenden inneren Bereich 12' von dem elektrisch leitenden Zwischenbereich 13' elektrisch trennt. Mit dieser Anordnung kann beispielsweise erreicht werden, dass der innere Bereich 12' einen Strom zur Stimulation von Körperzellen in das Körpergewebe überträgt und der Zwischenbereich 13' zum Abgriff von elektrischen Signalen der Körperzellen einsetzbar ist. Alternativ können beide elektrisch leitenden Bereiche 12', 13'jeweils einen Pol einer bipolaren Elektrodenspitze 10 bilden.

Alternativ kann der äußere Bereich 11' elektrisch leitend ausgebildet sein und vom Zwischenbereich 13' durch einen weiteren Bereich getrennt sein.

Fig. 5 zeigt eine Elektrodenspitze 10, die eine im Wesentlichen zylindrische Form aufweist. Die Elektrodenspitze 10 umfasst mehrere Materialschichten 11, 12, wobei die innere Schicht 12 einerseits den Kern der Elektrodenspitze 10 bildet und andererseits teilweise in die äußere Schicht 11 eingebettet ist. Die innere Schicht 12 umfasst ein elektrisch leitendes Material. Auf den Kern, der durch die innere Schicht 12 gebildet ist, kann ein isolierendes Material gesputtert bzw. abgeschieden werden, das bei dem Ausführungsbeispiel gemäß Fig. 5 die äußere Schicht 11 bildet. In einem weiteren Schritt wird auf das isolierende Material der äußeren Schicht 11 eine weitere innere Schicht 12 gesputtert, wobei die innere Schicht 12 strukturiert wird. Dabei ergeben sich auf dem Umfang der isolierenden äußeren Schicht 11 beabstandet angeordnete Bereiche der inneren Schicht 12, die im Wesentlichen in Längsrichtung der Elektrodenspitze 10 ausgerichtet sind. In einem weiteren Schritt wird die äußere Schicht weiter abgeschieden, so dass die Lücken zwischen den auf dem Umfang verteilten Bereichen der inneren Schicht 12 geschlossen und die innere Schicht 12 vollständig von der äußeren Schicht 11 ummantelt wird. Ingesamt umgibt die äußere Schicht 11 also einerseits den durch die innere Schicht 12 gebildeten Kern der Elektrodenspitze 10 und andererseits die separaten, vom Kern radial beabstandeten Bereiche, die ebenfalls durch die innere Schicht 12 gebildet sind, vollständig. Die vom Kern radial beabstandeten Bereiche der inneren Schicht 12 bilden im Wesentlichen einen strukturierten Ring 19. Das bedeutet, dass die vom Kern beabstandeten Bereiche der inneren Schicht 12 eine hohlzylindrische Form aufweisen, wobei die hohlzylindrische Form durch Öffnungen, Poren, Löcher oder Schlitze durchbrochen ist. Der strukturiere Ring 19 weist daher Lücken auf.

Es ist möglich, dass die radialen Außenflächen 17 der durch die innere Schicht 12 gebildeten, in Umfangsrichtung voneinander beabstandeten Bereiche freiliegen. Das bedeutet, dass die radialen Außenflächen 17 nicht durch die äußere Schicht 11 bedeckt sind. Vielmehr können die radialen Außenflächen 17 auf dem Außenumfang der Elektrodenspitze 10 angeordnet sein, so dass über die Außenoberfläche der Elektrodenspitze 10 elektrische Signale erfasst oder abgegeben werden können. Die isolierende äußere Schicht 11 füllt in diesem Fall den Zwischenraum zwischen dem Kern 18 und dem strukturierten Ring 19 sowie die Lücken im strukturierten Ring 19 aus. Der strukturierte Ring 19 bildet mit den ausgefüllten Lücken die Außenoberfläche der Elektrodenspitze 10.

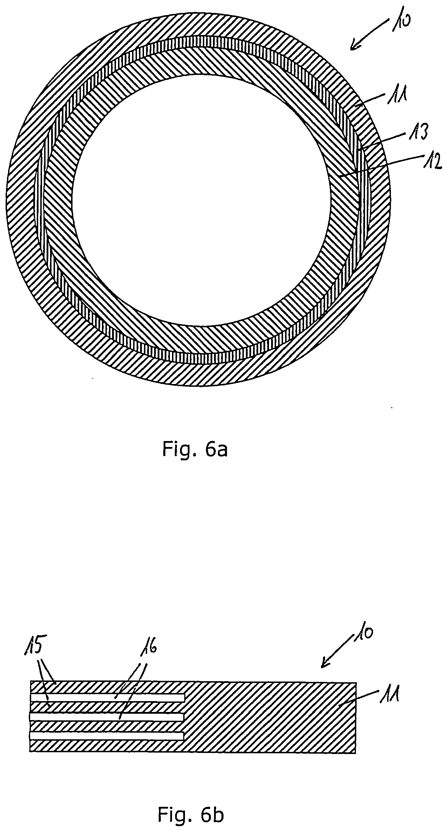

Die Elektrodenspitze 10 kann auch hohlzylindrisch geformt bzw. ausgebildet sein, wie in Fig. 6a dargestellt. Der Hohlzylinder, der die Tragstruktur der Elektrodenspitze 10 bildet, weist eine äußere Schicht 11, eine innere Schicht 12 und eine Zwischenschicht 13 auf, die sich zwischen der äußeren Schicht 11 und der inneren Schicht 12 erstreckt. Die äußere Schicht 11, die innere Schicht 12 und die Zwischenschicht 13 sind zueinander konzentrisch angeordnet. Die innere Schicht 12 begrenzt einen Innendurchmesser der Elektrodenspitze 10. Die äußere Schicht begrenzt einen Außendurchmesser der Elektrodenspitze 10. Die äußere Schicht 11 weist vorzugsweise ein Formgedächtnismaterial auf. Die innere Schicht 12 weist vorzugsweise ein Material auf, das eine höhere Leitfähigkeit umfasst, als die äußere Schicht 11. Umgekehrt kann die innere Schicht 12 durch einen Formgedächtniswerkstoff gebildet sein. Die äußere Schicht 11 kann eine erhöhte Leitfähigkeit aufweisen. Die Zwischenschicht 13 ist vorzugsweise aus einem isolierenden Material.

Es ist auch möglich, dass alle Schichten elektrisch leitend ausgebildet sind und durch Trennmaterialien getrennt sind.

Die mehrschichtig durch das Sputterverfahren bzw. allgemein Abscheideverfahren hergestellte Elektrodenspitze 10 kann anschließend strukturiert werden. Beispielsweise kann durch ein laserschneidendes Verfahren die hohlzylindrische Elektrodenspitze 10 gemäß Fig. 6a mit Aussparungen 16 versehen werden, wie in Fig. 6b dargestellt. Insbesondere können im Bereich eines axialen Endes der Elektrodenspitze 10 mehrer Aussparungen 16 eingebracht werden, so dass sich Arme 15 bilden. Die Aussparungen 16 weisen vorzugsweise über den Umfang der Elektrodenspitze 10 einen gleichmäßigen Abstand und eine gleichmäßige Breite auf. Dadurch ergeben sich gleichmäßig breite und voneinander beabstandete Arme 15, die beispielsweise zur Verankerung der Elektrodenspitze 10 im Körpergewebe einsetzbar sind. Die Arme 15 können in Längsrichtung der Elektrodenspitze 10 geradlinig verlaufen. Es ist auch möglich, dass die Arme 15 gekrümmt, insbesondere in radialer Richtung umgeformt sind. Die Arme 15 können auch in Umfangsrichtung der Elektrodenspitze 10 gebogen sein. Insbesondere können die Arme 15 spiralförmig um die Längsachse der Elektrodenspitze 10 gewunden, insbesondere spiralförmig gewunden, sein.

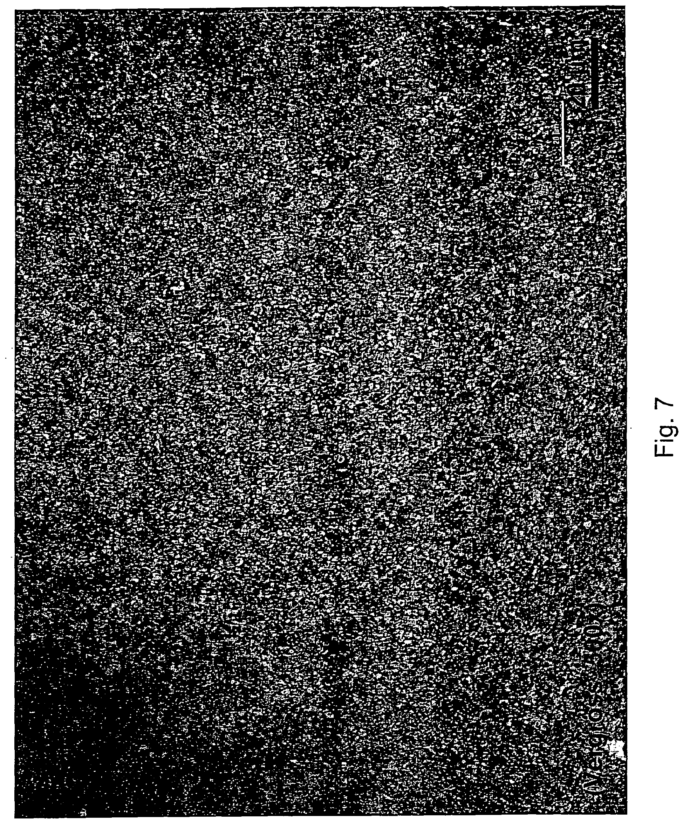

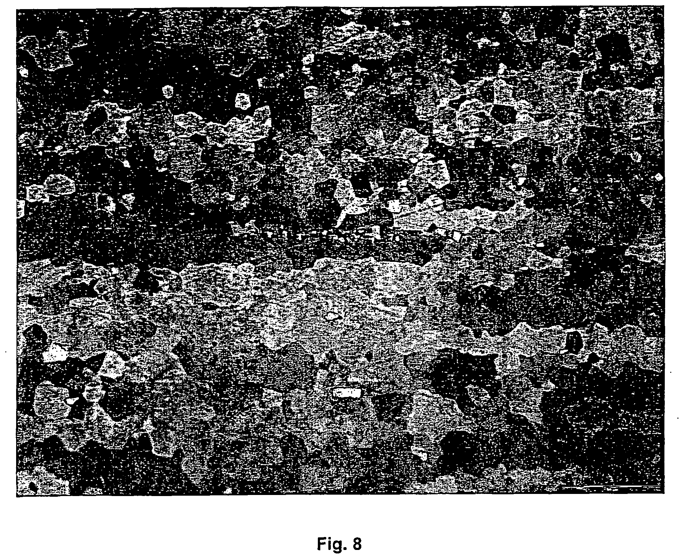

Fig. 7 verdeutlicht die kleinen Korngrößen, die im Vergleich zu herkömmlich hergestellten Elektrodenspitzen (Fig. 8) auf der Abscheidungsroute erhältlich sind. Außerdem ist die gleichmäßige Verteilung der Körner (Fig. 7) im Gegensatz zur zeilenförmigen Anordnung der Körner (Fig. 8) zu erkennen.

Alle Merkmale der vorstehend genannten Ausführungsbeispiele, die im Zusammenhang mit der Elektrode bzw. der Elektrodenspitze offenbart sind, werden auch im Zusammenhang mit dem Verfahren zur Herstellung der Elektrode bzw. der Elektrodenspitze offenbart und umgekehrt. Ferner können alle einzelnen Merkmale der Ausführungsbeispiele untereinander kombiniert werden.

### Bezugszeichenliste

- 10: Elektrodenspitze
- 11: äußere Schicht
- 11': äußerer Bereich
- 12: innere Schicht
- 12': innerer Bereich
- 13: Zwischenschicht
- 13': Zwischenbereich
- 14: Trennschicht
- 14': Trennbereich
- 15: Arm
- 16: Aussparung
- 17: radiale Außenfläche
- 18: Kern
- 19: strukturierter Ring

## Patentansprüche

1. Elektrode zur Messung elektrischer Aktivitäten eines lebenden Körpers und/oder zur Abgabe elektrischer Signale in den lebenden Körper und/oder zur Ablation von Gewebe oder Nervenbahnen mit einer elektrisch isolierten, flexiblen Leitung, die mit einer distal angeordneten, elektrisch leitenden Elektrodenspitze (10) fest oder lösbar verbunden ist, wobei die Elektrodenspitze (10) zur Einfuhr in den Körper angepasst ist,
**dadurch gekennzeichnet, dass**
zumindest eine Tragstruktur der Elektrodenspitze (10) wenigstens einen kristallinen Formgedächtniswerkstoff umfasst und die Elektrodenspitze (10) auf Grund des Formgedächtniswerkstoffes flexibel ist, wobei der Formgedächtniswerkstoff der Tragstruktur zumindest teilweise durch Sputtern oder durch lasergestützte Abscheidung gebildet ist derart, dass durch Variation einzelner Prozessparameter, insbesondere des Kammerdrucks und/oder des Abscheidedrucks und/oder des Partialdrucks und/oder der Targetzusammensetzung, Ausscheidungen im Formgedächtniswerkstoff eine maximale Größe von 500 nm aufweisen.

2. Elektrode nach Anspruch 1,
**dadurch gekennzeichnet, dass**
der Formgedächtniswerkstoff der Tragstruktur eine durch Sputtern oder durch lasergestützte Abscheidung gezielt eingestellte maximale Dichte an Materialinhomogenitäten, wie Gitterbaufehler aufweist.

3. Elektrode nach Anspruch 1 oder 2,
**dadurch gekennzeichnet, dass**
die Korngröße des Formgedächtniswerkstoffs der Tragstruktur weniger als 4 µm, insbesondere weniger als 3 µm, insbesondere weniger als 2 µm, insbesondere weniger als 1,5 µm, insbesondere weniger als 1 µm, insbesondere weniger als 0,5 µm, insbesondere weniger als 250 nm, beträgt.

4. Elektrode nach wenigstens einem der vorhergehenden Ansprüche,
**dadurch gekennzeichnet, dass**
der Formgedächtniswerkstoff eine Nickel-Titan-Legierung umfasst, insbesondere mit einer Zusammensetzung aus 50,8 at.% Nickel und 49,2 at.% Titan.

5. Elektrode nach wenigstens einem der Ansprüche 1 bis 4,
**dadurch gekennzeichnet, dass**
der Formgedächtniswerkstoff eine ternäre oder quaternäre Legierung umfasst.

6. Elektrode nach wenigstens einem der vorhergehenden Ansprüche,
**dadurch gekennzeichnet, dass**
die Elektrodenspitze (10) einen mehrschichtigen Aufbau aufweist.

7. Elektrode nach Anspruch 6,
**dadurch gekennzeichnet, dass**
eine radial äußere Schicht (11) oder mehrere radial äußere Schichten aus dem Formgedächtniswerkstoff, insbesondere aus einer Nickel-Titan-Legierung, und eine radial innere Schicht (12) oder mehrere radiale innere Schichten aus einem Material mit höherer elektrischer Leitfähigkeit als das Material der radial äußeren Schicht (11) oder radial äußeren Schichten bestehen, insbesondere aus Kupfer, Silber, Gold, Platin, Tantal, Niob, Palladium oder Kohlenstoff.

8. Elektrode nach Anspruch 6 oder 7,
**dadurch gekennzeichnet, dass**
zwischen den Schichten (11, 12) ein in radialer Richtung kontinuierlicher Übergang besteht.

9. Elektrode nach wenigstens einem der Ansprüche 1 bis 8,
**dadurch gekennzeichnet, dass**
die radial innere Schicht (12) oder die radiale inneren Schichten aus einer Nickel-Titan-Legierung und die radial äußere Schicht (11) oder die radial äußeren Schichten aus einem Material mit höherer elektrischer Leitfähigkeit als das Material der radial inneren Schicht (12) oder radial inneren Schichten hergestellt sind.

10. Verfahren zur Herstellung einer Elektrode zur Messung elektrischer Aktivitäten eines lebenden Körpers und/oder zur Abgabe elektrischer Signale in den lebenden Körper mit einer elektrisch isolierten, flexiblen Leitung, die mit einer distal angeordneten, elektrisch leitenden Elektrodenspitze (10) fest oder lösbar verbunden ist, wobei die Elektrodenspitze (10) zur Einfuhr in den Körper angepasst ist,
**dadurch gekennzeichnet, dass**
zumindest eine Tragstruktur der Elektrodenspitze (10) aus wenigstens einem kristallinen Formgedächtniswerkstoff zumindest teilweise durch Sputtern oder durch lasergestützte Abscheidung gebildet wird derart, dass die Elektrodenspitze (10) auf Grund des Formgedächtniswerkstoffes flexibel ist, wobei durch Variation einzelner Prozessparameter, insbesondere des Kammerdrucks und/oder des Abscheidedrucks und/oder des Partialdrucks und/oder der Targetzusammensetzung, Ausscheidungen im Formgedächtniswerkstoff eine maximale Größe von 500 nm aufweisen.

11. Verfahren nach Anspruch 10,
**dadurch gekennzeichnet, dass**
mehrere Schichten (11, 12) aus unterschiedlichen Materialien zur Bildung der Elektrodenspitze (10) aufgebracht werden, wobei die radial inneren Schichten (12) und die radial äußeren Schichten (11) durch eine Kombination aus einem Lithographieverfahren und einem Sputterverfahren hergestellt werden.

## Claims

1. Electrode for measuring electrical activities of a living body and/or emitting electrical signals into the living body and/or ablating tissue or nerve paths with an electrically insulated, flexible line which is connected fixedly or detachably to a distally arranged, electrically conductive electrode tip (10), wherein the electrode tip (10) is designed for insertion into the body, **characterised in that** at least one support structure of the electrode tip (10) comprises at least one crystalline shape memory material, and the electrode tip (10) is flexible due to the shape memory material, wherein at least part of the shape memory material of the support structure is formed by sputtering or by laser-assisted deposition, in such manner that the size of precipitations in the shape memory material can be limited to not more than 500 nm by varying individual process parameters, particularly the chamber pressure and/or the precipitation pressure and/or the partial pressure and/or the target composition.

2. Electrode according to claim 1, **characterised in that** the shape memory material of the support structure has a maximum density of material inhomogeneities, such as lattice defects, adjusted in targetted manner by sputtering or by laser-assisted deposition.

3. Electrode according to claim 1 or 2,**characterised in that** the grain size of the shape memory material of the support structure is less than 4 µm, particularly less than 3 µm, more particularly less than 2 µm, most particularly less than 1.5 µm, especially less than 1 µm, more especially less than 0.5 µm, most especially less than 250 nm.

4. Electrode according to at least one of the preceding claims, **characterised in that** the shape memory material comprises a nickel-titanium alloy, particularly with a composition of 50.8 at.% nickel and 49.2 at.% titanium.

5. Electrode according to at least one of claims 1 to 4, **characterised in that** the shape memory material comprises a ternary or quaternary alloy.

6. Electrode according to at least one of the preceding claims, **characterised in that** the electrode tip (10) has a multilayer structure.

7. Electrode according to claim 6, **characterised in that** one or more radially outer layer(s) (11) consist(s) of the shape memory material, particularly of a nickel-titanium alloy, and one or more radially inner layer(s) (12) consist(s) of a material with greater electrical conductivity than the material of the one or more radially outer layer(s) (11), particularly of copper, silver, gold, platinum, tantalum, niobium, palladium or carbon.

8. Electrode according to claim 6 or 7, **characterised in that** a radially continuous transition is present between the layers (11, 12).

9. Electrode according to at least one of claims 1 to 8, **characterised in that** the one or more radially inner layers (12) consist of a nickel-titanium alloy and the one or more radially outer layers (11) consist of a material with greater electrical conductivity than the material of the one or more radially inner layers.

10. Method for fabricating an electrode for measuring electrical activities of a living body and/or emitting electrical signals into the living body with an electrically insulated, flexible line which is connected fixedly or detachably to a distally arranged, electrically conductive electrode tip (10), wherein the electrode tip (10) is adapted for insertion into the body, **characterised in that** at least a part of at least one support structure of the electrode tip (10) is formed from at least one crystalline shape memory material by sputtering or laser-assisted deposition, in such manner that the electrode tip (10) is flexible due to the shape memory material , wherein the precipitations in the shape memory material can be limited to not more than 500 nm by varying individual process parameters, particularly the chamber pressure and/or the precipitation pressure and/or the partial pressure and/or the target composition.

11. Method according to claim 10, **characterised in that** several layers (11, 12) of different materials are applied to form the electrode tip (10), wherein the radially inner layers (12) and the radially outer layers (11) are produced by a combination of a lithography method and a sputtering method.

## Revendications

1. Electrode pour la mesure d'activités électriques d'un corps vivant et/ou pour l'émission de signaux électriques dans le corps vivant et/ou pour l'ablation de tissus ou de voies nerveuses avec un câble flexible électriquement isolé, qui est relié fixement ou de manière amovible, avec une pointe d'électrode électroconductrice disposée distalement, la pointe d'électrode (10) étant adaptée pour l'introduction dans le corps, **caractérisée en ce qu'**au moins une structure de support de la pointe d'électrode (10) comprend au moins un matériau à mémoire de forme cristallin et la pointe d'électrode (10) est flexible sur la base du matériau à mémoire de forme, le matériau à mémoire de forme de la structure de support est au moins en partie formé par pulvérisation ou par déposition assistée au laser de telle manière que par variation des paramètres de processus individuels, notamment de la pression de chambre et/ou de la pression de déposition et/ou de la pression partielle et/ou de la composition cible, les dépôts dans le matériau à mémoire de forme présentent une taille maximale de 500 nm.

2. Electrode selon la revendication 1, **caractérisée en ce que** le matériau à mémoire de forme de la structure de support comporte une densité en inhomogénéités matérielles maximale, comme des défauts de réseaux, établie de façon appropriée par pulvérisation ou par déposition assistée au laser.

3. Electrode selon la revendication 1 ou 2, **caractérisée en ce que** la granulométrie du matériau à mémoire de forme de la structure de support est inférieure à 4 µm, notamment inférieure à 3 µm, notamment inférieure à 2 µm, notamment inférieure à 1,5 µm, notamment inférieure à 1 µm, notamment inférieure à 0,5 µm, notamment inférieure à 250 nm.

4. Electrode selon l'une quelconque des revendications précédentes, **caractérisée en ce que** le matériau à mémoire de forme comprend un alliage nickel-titane, notamment avec une composition de 50,8 at. % de nickel et 49,2 at. % de titane.

5. Electrode selon l'une quelconque des revendications 1 à 4, **caractérisée en ce que** le matériau à mémoire de forme comprend un alliage ternaire ou quaternaire.

6. Electrode selon l'une quelconque des revendications précédentes, **caractérisée en ce que** la pointe d'électrode (10) comporte une structure multicouche.

7. Electrode selon la revendication 6, **caractérisée en ce qu'**une couche radialement extérieure (11) ou plusieurs couches radialement extérieures sont en matériau à mémoire de forme, notamment dans un alliage nickel-titane, et une couche radialement intérieure (12) ou plusieurs couches radialement intérieures dans un matériau présentant une électro-conductivité plus élevée que le matériau de la couche radialement extérieure (11) ou des couches radialement extérieures, notamment en cuivre, argent, or, platine, tantale, niobium, palladium ou carbone.

8. Electrode selon la revendication 6 ou 7, **caractérisée en ce qu'**entre les couches (11,12) il y a un transfert continu dans le sens radial.

9. Electrode selon au moins l'une quelconque des revendications 1 à 8, **caractérisée en ce que** la couche radialement intérieure (12) ou les couches radialement intérieures sont fabriquées dans un alliage nickel-titane et la couche radialement extérieure (11) ou les couches radialement extérieures dans un matériau avec une électro-conductivité plus élevée que le matériau de la couche radialement intérieure (12) ou les couches radialement intérieures.

10. Procédé pour la fabrication d'une électrode pour la mesure d'activités électriques d'un corps vivant et/ou pour l'émission de signaux électriques dans le corps vivant avec un câble flexible électriquement isolé, qui est relié, fixement ou de manière amovible, avec une pointe d'électrode électroconductrice (10) disposée distalement, la pointe d'électrode (10) étant adaptée pour l'introduction dans le corps, **caractérisé en ce qu'**au moins une structure de support de la pointe d'électrode (10) en au moins un matériau à mémoire de forme cristallin est formée au moins en partie par pulvérisation ou par déposition assistée au laser de telle manière que la pointe d'électrode (10) est flexible sur la base du matériau à mémoire de forme, par variation des paramètres de processus individuels, notamment de la pression de chambre et/ou de la pression de déposition et/ou de la pression partielle et/ou de la composition cible, les dépôts dans le matériau à mémoire de forme présentent une taille maximale de 500 nm.

11. Procédé selon la revendication 10 **caractérisé en ce que** plusieurs couches (11,12) en matériaux différents sont appliquées pour former la pointe d'électrode (10), les couches radialement intérieures (12) et les couches radialement extérieures (11) étant fabriquées en combinant un procédé lithographique et un procédé de pulvérisation.
